# EUROPEAN PATENT APPLICATION

(11) **EP 1 847 223 A1**
(43) Date of publication of application: **24.10.2007**
(21) Application number: 06008117.1
(22) Date of filing: 19.04.2006
(51) Int. Cl.: A61B 17/04, A61B 19/00

(54) **Actuator for minimally invasive surgery**

(71) Applicant: Mehmanesh, Hormoz, Tehran (IR)
(72) Inventor: Mehmanesh, Hormoz, Tehran (IR)
(74) Representative: Grünecker, Kinkeldey, Stockmair & Schwanhäusser Anwaltssozietät

(57) **Abstract**

In a surgery device for minimally invasive surgery, an actuator system is provided which causes the actuator components to perform a specified sequence of motions in order to fulfill a predefined surgical task. The sequence of predefined motions may be initiated by a respective control sequence implemented in a respective control system which may be provided in the form of a programmable interface, thereby imparting a certain degree of intelligence to the actuator system. Consequently, accuracy of the corresponding surgical task may be significantly enhanced since human related imperfections may be significantly reduced. Moreover, a method is provided in which a strategy is described for implementing a sequence of motions into an actuator system so as to accomplish a predefined task in endoscopic surgery.

## Description

Generally, the present invention relates to techniques for minimally invasive surgery, in which the operation region is accessed by one or more appropriate access canulas on the basis of endoscopic devices.

In the vast field of surgery, minimally invasive surgery is increasingly gaining in importance, due to its advantages with respect to efforts in terms of instruments and convalescence of the patient, which directly translates into respective cost savings for the entire treatment. In minimally invasive surgery techniques, small and precise hand tools are typically used, which are inserted into the operation region by respective access canula. Consequently, performing surgery tasks on the basis of endoscopic techniques may require special mechanical skills, since some of these surgery tasks are difficult to be accomplished by means of these small hand tools. For example, missing the depth of operation, unintended movements of the surgeon's hands, which are directly transferred to the end-effector of the surgery device via a respective actuator mechanism, and the like, are typical problems that have to be dealt with in order to achieve satisfactory surgery results. Consequently, also in this field of surgical techniques it is attempted to perform pre-defined motions on the basis of an automated robotic system, in order to increase the precision of the surgery and try to avoid, or at least reduce, some of the inaccuracies introduced by human imperfections.

In the field of minimally invasive surgery, the task of providing a suture is one of the most sophisticated processes. Although, in conventional robotic systems, a complex mechanism with actuators and motion transfer mechanisms is provided so as to directly transfer the surgeon's hand motions to the robot arms of the surgery device, forming a suture, for instance a single knot or even a running suture, is nevertheless a highly complex task and may nevertheless require a high degree of mechanical skill on the side of the respective surgeon. Furthermore, conventional robotic systems, transferring the surgeon's hand motions into the corresponding operation region, may typically require at least two access canula, thereby increasing the degree of invasion of the surgery. As a consequence, in the conventional endoscopic surgery technique performing suture-related tasks is a highly sophisticated process demanding a high degree of skill and concentration from the surgeon, while on the other side prolonged treatment time and increased degree of invasiveness place an increased burden on the patient.

In view of the situation described above, there is a need for an enhanced technique, in order to perform suture related surgery tasks in a more efficient manner.

According to one aspect of the present invention, the object is solved by an endoscopic surgery device for minimal invasive surgery. The device comprises end-effector means configured to be moveable in an operation region. Moreover, the device further comprises an actuator system, mechanically coupled to the end-effector means and configured for automatically effecting a pre-defined sequence of actuator actions, to actuate the end-effector means for performing a pre-defined sequence of motions for forming at least a portion of a suture.

Consequently, the inventive surgery device is configured to automatically perform a sequence of motions for forming a suture or at least a portion thereof, thereby achieving a high degree of precision while significantly relaxing the constraints for a surgeon by using the benefits of robotic technologies of conventional surgical systems, wherein, however, based on the actuator system of the present invention, a sophisticated surgical task may be accomplished in a highly automated manner. In conventional minimal invasive surgical techniques, a suturing procedure is performed by a surgeon by using a couple of needle holders, wherein, in conventional robotic systems, the surgeon's hand motions are directly transferred to the robotic arms in order to convert the hand motions into substantially the same motions of the end-effectors of the robotic arms, for providing a suture. In this case, however, as previously explained, the requirement for a high degree of mechanical abilities is imposed on the surgeon, wherein, nevertheless, substantially unavoidable human constraints, such as hand shaking, and the like, may restrict the precision achievable by conventional techniques. Consequently, with the inventive actuator system having the capability for automatically effecting a pre-defined sequence of actuator actions, so as to perform a suture related task, unintended or non-controllable movements of the respective end-effector means of conventional robotic systems may substantially be avoided, thereby significantly increasing the degree of precision. Moreover, a suture-related task may be accomplished in a more time efficient manner, thereby also relaxing the constraints for the medical staff and for the patient.

In an advantageous embodiment, the end effector means comprises a gripping system. Consequently, conventional suture needles may efficiently be used in combination with the present invention, thereby providing for a high degree of compatibility with well-approved surgical techniques and instruments.

In a further embodiment, the gripping system comprises a first gripper and a second gripper. Consequently, a conventional suture needle may be operated, on the basis of the first and second grippers, wherein, advantageously, a single access canula may be sufficient for introducing the first and second grippers into the operation region and to perform the pre-defined sequence of motions, thereby significantly reducing the degree of invasiveness of the respective surgery process.

In a further advantageous embodiment, at least one of the first and second grippers is configured to be moveable with respect to two or more degrees of freedom. Consequently, even moderately complex suture-related motions may be performed by the first and the second gripper elements, thereby allowing a high degree of freedom that is similar to the required degree of freedom of the surgeon's hands, while nevertheless avoiding any non-controlled movements of the first and second gripper elements.

In one illustrative embodiment, at least one of the two or more degrees of freedom corresponds to a rotational motion around a specified axis. Thus, the end-effector means of the present invention is configured to perform any motions related to performing a circle or a portion thereof, which may be required for tying a knot or forming a running suture. In a further embodiment, at least one of the two or more degrees of freedom corresponds to a linear motion along the specified axis. Consequently, the rotational motion may be displaced along the specified rotational axis, thereby allowing for a corresponding linear displacement of the suture needle, with respect to a previous center of rotation. Hence, a running suture may be accomplished, or the required linear displacements may be provided for forming a single knot.

According to a further illustrative embodiment, the device further comprises a control unit operatively connected to the actuator system, wherein the control unit is configured to control the pre-defined sequence of actuator actions. In some embodiments, the control unit may itself be configured to create respective control signals for the actuator system in order to effect respective actuator components in the system so as to perform the required sequence of motions. In still other cases, the control unit may be configured for "supervising" the activity of the actuator system, having implemented therein an algorithm for automatically performing the specified sequence of actuator actions, thereby providing for an enhanced degree of precision and safety, since the control unit may detect any deviation from a pre-defined routine and may, depending on the configuration of the control unit, respond by means of appropriate interaction. In still other illustrative embodiments, the control unit may be configured to perform real-time calculations with respect to the pre-defined sequence of motions, for instance on the basis of a corresponding model and / or feed forward data and / or feedback data, in order to provide corresponding control signals to the actuator system for effecting the required sequence of actuator action. Thus, in some illustrative embodiments, the control unit may represent a portion of the actuator system, having implemented therein a corresponding routine for instructing the actuator system to perform the required sequence of actions, while in other illustrative embodiments, the control unit may act as an appropriate interface for respective components of the actuator system, in order to provide thereto respective commands for performing the required sequence of actuator action. In this case, a further internal or external source may be provided, which may transmit respective commands, resulting in the desired activity of the actuator system.

In a further advantageous embodiment, the control unit is configured to obtain position data for controlling the sequence of actuator actions, on the basis of the position data. Consequently, a high degree of precision may be accomplished by using the position data which may be provided by any appropriate external or internal source, such as, for instance, position sensors provided within the actuator system and / or within the end-effector means, or by any other external source, such as a monitoring system, on the basis of respective image data and the like. In this case, the control unit may appropriately coordinate the actuator actions, on the basis of the position data, thereby allowing a high degree of adaptation to specific operation conditions, imparting a high degree of flexibility to the inventive surgery device.

In a further illustrative embodiment, the control unit is configured to control the sequence of actuator actions, on the basis of the position data and at least one target value, for at least some of the actions of the sequence. In this case, an effective feedback control mechanism may be provided, wherein the corresponding position data may be compared to a respective target value, in order to obtain a high degree of precision. For instance, corresponding position data may be obtained from motor encoders of the actuator system, from specifically designed position detectors, and the like, in order to precisely position the respective actuator means and thus the end-effector means, on the basis of the target value. In still other illustrative embodiments, the position data may be provided in the form of image data, such as visual inspection data, ultra-sonic data, x-ray data, and the like, which may, after an appropriate image data processing, be compared with respective target values, in order to accomplish a high degree of precision. Similarly, additionally or alternatively, an effective feed forward control mechanism may be provided, on the basis of respective position data and a target value, for instance by basing one ore more parameter values of one or more of the next actuator actions, such as the length of a linear displacement, and the like, upon respective position data so as to adaptively perform the one or next procedural steps of the pre-defined sequence of motions.

In a further illustrative embodiment, the actuator system comprises one or more position sensors for providing the position data. As previously discussed, the utilization of respective position sensors may provide for enhanced control accuracy in respective feed forward and / or feed back control strategies.

In one illustrative embodiment, the control unit is configured to generate a plurality of control signals for driving the actuator system, for effecting the pre-defined sequence of actuator actions. Hence, as previously explained, the control unit may serve as a source of appropriate control signals and thus act as a part of the inventive actuator system, in order to achieve the required suture related surgical task. In combination with the ability of receiving position data and responding thereto, the surgical device may provide for a high degree of precision and flexibility, as was previously described.

In a further embodiment, the control unit comprises means for updating the pre-defined sequence of actuator actions. In this case, additionally or alternatively to providing basic control signals or providing closed loop control strategies, the control unit may have the capability of significantly altering the pre-defined sequence of actuator actions. That is, other than in the previously described case of enhanced control, wherein a given basic actuator action may be "adapted" with respect to position data and the like, updating of the pre-defined sequence may enable the surgery device to significantly alter one or more of the actions, in order to enable the replacement of the end effector means to to accomplish the same or a different surgical task on the basis of the updated sequence. In still other cases the end effector means may have a plurality of degrees of freedom, thereby allowing a plurality of different tasks to be performed, wherein the updating means, which may be provided in the form of a memory device, enables the "uploading" of an appropriate instruction sequence for effecting an appropriate sequence of motions.

In a further advantageous embodiment, the means for updating is configured to obtain process information related to a surgical task performed or to be performed by the endoscopic surgery device, and to update the predefined sequence of actuator actions on the basis of the process information. While, in the previous cases, updating of the predefined sequence may result in the implementation of a different type of sequence, independently from previously performed surgical tasks, in the present embodiment the process of updating may be performed on the basis of process information that corresponds to the surgical tasks previously performed or still to be performed by the device. Consequently, the respective process information may be used for enhancing the accuracy of a device by implementing a "learn" phase during which previously used parameters in performing the actuator actions may be redefined so as to produce a result with enhanced accuracy, and / or the process information may specify a specific type of suture process requiring a specified updated version of the initially implemented process sequence. For instance, internally or externally generated process information may be transferred to the control unit, for instance by wired or wireless interfaces, in order to provide an appropriate set of control signals for obtaining the desired updated sequence of actuator actions. In this way, the control unit may be reprogrammed for enhancing accuracy or for performing a different task.

In some illustrative embodiments, the process information may include a corresponding specific updated instruction set for creating respective control signals for performing the updated sequence of actuator action. For example, if the control unit is configured to provide drive signals for the actuator components, without having implemented therein a complete set of control signals require for performing the surgical task, a required instruction set or an appropriate set of control signals for effecting the desired updated version of the entire sequence or corresponding data may be obtained from an external source, for instance a supervising control system, an operator based external data source and the like, in order to provide the updated sequence for the surgical task to be performed. In this case, the control unit may act as an interface for receiving and converting instructions and data and forwarding appropriate control signals to the various actuator components.

In still another illustrative embodiment, the device may comprise a data communications interface for communication with an external source. As previously already indicated, process related data, such as process information, position data, updates for an instruction set, and the like, may be provided by an external source, thereby allowing the provision of high computational and data storing capabilities, that are available for the device by means of an external source, while still maintaining the required installation volume for a respective control unit in the device at a very low level. Moreover, providing a respective data communications interface, which may, in some advantageous embodiments, be implemented as a wireless interface, provides for a high degree of flexibility, since communication to other devices, such as other surgical devices, computer means, a supervising control system, independent position data and other sensor systems and the like, may readily be established. For example, the results of a corresponding suture related activity of the surgical device may be analyzed and assessed by an external system, on the basis of respective data obtained by the device and communicated to the external system by means of the data communications interface.

In some illustrative embodiments, the data communications interface is configured to provide real-time data communication between the external source and the actuator system. Based on this capability, the control capacity of the actuator system and thus volume and constructional requirement may be maintained at a low level, since corresponding control systems for effecting the required actuator actions may be supplied from the external source, thereby providing the possibility of using a high computational and data storing capacity of an external source, while nevertheless achieving a substantially "instantaneous" response of the actuator components to the external source. Consequently, the actuator system may have implemented therein merely corresponding actuator control components, similarly as in conventional robotic systems, wherein the corresponding instructions for coordinating the respective actuator components controllers are obtained via the data communications interface.

In one illustrative embodiment, the device further comprises a housing, including a handle, wherein the handle is configured to provide for one hand operation of the surgery device. Thus, in addition to avoiding an additional access to the operation region, the design of the housing of the device may significantly facilitate its operation by the surgeon while, additionally, increased flexibility is achieved, since the surgeon may operate a further device such as an inspection device and the like, while performing a highly complex suture-related task.

In a further illustrative embodiment, the device further comprises a first holder, coupling the first gripper to the actuator system, and a second holder coupling the second gripper to the actuator system, wherein the first and the second holders are arranged in a co-axial relationship. Due to this configuration of the device, a spatially efficient construction may be achieved, wherein the complex sequence of suture related motions may be performed on the basis of the co-axially arranged holders.

In a further illustrative embodiment, the gripping means is configured to support different types of suture needles, thereby providing a high degree of flexibility and compatibility with conventional minimally invasive surgery techniques.

According to a further aspect of the present invention, the above-described object is solved by a method comprising the determination of a first sequence of motions of an end-effector system of an endoscopic surgery device for a first suture related surgery task. The method further comprises implementing a first control sequence into a control unit for controlling an actuator system for effecting the first sequence of motions. Consequently, a desired suture-related surgical task may efficiently be performed by an automated system, in that a respective control sequence is implemented in an appropriately configured actuator system. Hence, the accuracy of complex suture-related surgical tasks may significantly be increased, while many of the human related restrictions may be avoided.

In a further advantageous embodiment, determining the first sequence of motions comprises analyzing the entire motion of a surgical instrument required to complete the first suture-related surgery task, and establishing a plurality of equivalent motions of the end-effector system for completing the first suture related surgery task using the surgical instrument. Hence, "mapping" the motion of a surgical instrument as used by a human operator on the basis of a respective endoscopic robotic system, into a plurality of equivalent motions of the end-effector system, a respective sequence of motions may be provided which may then be appropriately implemented into the actuator system and retrieved on demand, thereby eliminating the human interaction with the surgical instrument. Thus, as previously already explained, a significantly enhanced degree of accuracy may be achieved.

In a further illustrative embodiment, analyzing the entire motion of the surgical instrument comprises determining a degree of freedom for each of the plurality of equivalent motions. Upon recognizing the required degree of freedom of the equivalent motions, the characteristics of the respective actuator system may be appropriately selected and the mechanical construction thereof may be designed in accordance with the determined degree of freedom. Consequently, based on the respective results, the mechanical construction may be selected. In still other illustrative embodiments, the determined degree of freedom may be compared with the capability of an existing actuator system and if a non-compliance may be recognized, a different set of equivalent motions may be established which may then again be compared with the capabilities of the given actuator system. In this way, a plurality of different suture related surgical tasks may be realized on the basis of the same actuator system, thereby providing for enhanced flexibility and cost effectiveness.

In a further advantageous embodiment, the method further comprises obtaining process data relating to the first suture-related surgery task, accomplished by using the first control sequence and updating the first control sequence on the basis of the process data. In this way, the efficiency of a corresponding control sequence may be enhanced on the basis of the process data, wherein, in some illustrative embodiments, the process of updating may be performed in a substantially automatic manner, for instance, using adaptive systems, such as neuronal networks and the like, in order to adjust "free" parameters for controlling at least some of the motions on the basis of first control sequence. In still other illustrative embodiments, the process of updating may be performed on the basis of respective simulation calculations and models in which the result of the first control sequence may be assessed and corresponding adaptations may be implemented in order to obtain the updated version of the first control sequence, which may then again be assessed on the basis of simulation and / or experimentation and / or results of actual related suture-related surgical tasks. Consequently, in some illustrative embodiments, updating of the first control sequence may be performed via the control unit, while in other embodiments updating of the first control sequence may be performed by an external source, for instance, via a data communications interface.

In still a further illustrative embodiment, the method comprises implementing a second control sequence in the control unit for controlling the actuator system for effecting a second sequence of motions of the end-effector system for a second specified suture-related task. In this way, different surgical tasks may be performed by the end-effector system, thereby enhancing the flexibility and efficiency thereof. Thereby, the second control sequence may be established on substantially the same principles as explained above, that is, by analyzing the corresponding surgical task performed by a human surgeon and establishing a plurality of appropriate equivalent motions of the end-effector system. For this purpose, the equivalent motions may be analyzed with respect to their compatibility with the mechanical characteristics of the actuator system and the end-effector system, and may correspondingly be redesigned when a corresponding non-compatibility is recognized. In other cases, the corresponding equivalent motions for the first and second surgical tasks may be used as a basis for designing the corresponding actuator and end-effector system in order to provide the capability for independently performing the first and second surgical tasks. In some illustrative embodiments, the first and second control sequences may be implemented in parallel, so that both sequences are simultaneously resident in the control unit, wherein in other cases one control sequence may be replaced by the other, thereby reducing the required data storage in the control unit. Moreover, as previously explained, in some illustrative embodiments the control unit may be provided as an external source with respect to the corresponding actuator system and end-effector system, wherein the corresponding data communication may be performed via an appropriate data interface, wired or wireless, in order to instruct the actuator means to perform the first or second equivalent motions on the basis of the first or second control sequences.

Further illustrative embodiment, objects and advantages of the present invention, will be described in more detail in the following detailed description, which is provided in combination with the accompanying drawings, in which:
- Figures 1A to 1E schematically illustrate a sequence of steps for tying a knot as a first surgical task;
- Figures 1F to 1J schematically illustrate respective steps for forming a running suture as a second surgical task to be analyzed, in order to establish respective equivalent motions for an automated actuator system;
- Figure 2A schematically illustrates a set of equivalent motions and the corresponding degrees of freedom required to perform the surgical tasks shown in Figures 1A to 1J;
- Figure 2B schematically illustrates the degrees of motional freedom required for a corresponding end-effector system comprising a pair of grippers to perform the equivalent motions shown in Figure 2A;
- Figure 2C schematically illustrates a surgical device according to the present invention in a perspective view;
- Figure 2D schematically illustrates an enlarged view of an end-effector system of the device, as shown in Figure 2C;
- Figure 2E schematically illustrates an actuator system, including a programmable interface, for effecting a sequence of pre-defined actuator actions according to one illustrative embodiment of the present invention; and
- Figures 3A to 3H schematically illustrate a surgery device according to the present invention, assessed on the basis of a simulation.

With reference to the accompanying drawings further illustrative embodiments of the present invention will now be described in more detail. In these examples, a system of a suturing device, used for endoscopic surgery, is provided, wherein an actuator system is configured to provide, in an automated fashion, a corresponding control sequence so as to effect a required sequence of motions, in order to fulfill the desired suture-related task. During a suturing procedure in the minimal invasive surgery, the surgeon typically uses a couple of needle holders for making the complete suture. Via conventional robotic systems, each movement of the surgeon's hands is directly transferred to the robotic arms, which then provide corresponding motions in the operation region. In order to achieve the same result, however, in an automatic fashion, thereby saving time and improving accuracy, a respective end-effector device, i.e. respective manipulators, may be provided, having substantially the same degree of freedom compared to the surgeon's hands during a minimal invasive surgery task.

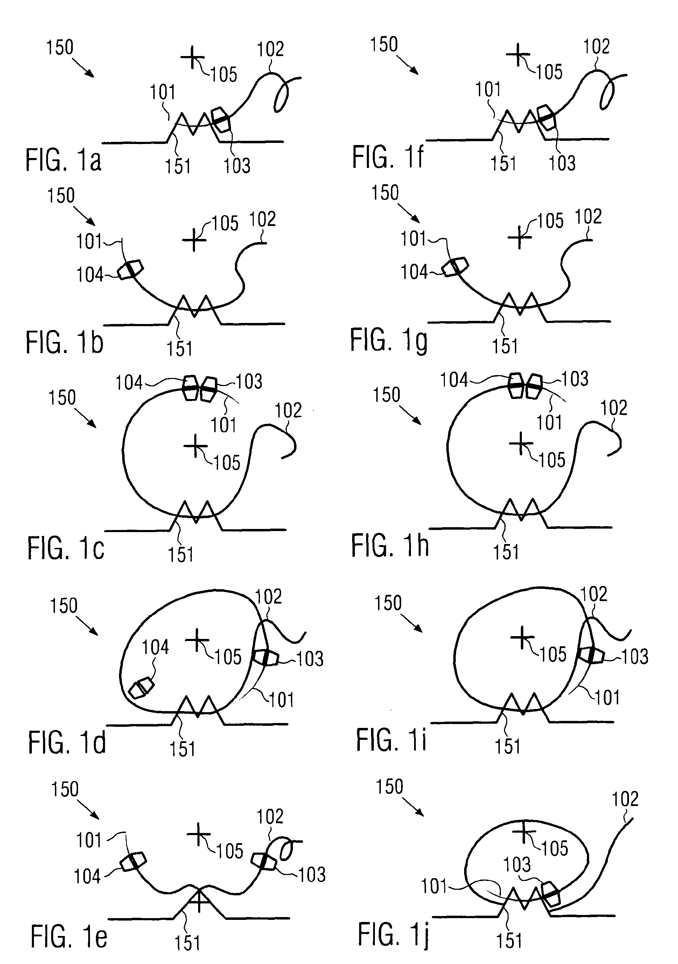

For instance, Figures 1A to 1E schematically illustrate respective process steps during the completion of a suture-related surgical task, which in this case corresponds to a single knot tying technique. For instance, in Figure 1A, and operation region 150 may include respective tissue portions 151 that are to receive a suture on the basis of a suture needle 101 and a corresponding thread material 102. At this stage of the process, the needle 101 may be supported by a respective gripper or manipulator 103, which may then be guided to and through the tissue 151 by appropriately moving the holder 103 on the basis of the respective movement of the surgeon's hand. In performing a single knottying process, a key factor is that the thread 102 may be placed over the holder 103, since then a loop can be formed by passing the needle 101 to another holder.

Figure 1B schematically shows the process in this situation, when the needle 101 is supported by a second or slave holder 104.

Figure 1C schematically illustrates the situation during the transfer of the needle 101 from the slave holder 104 to the master holder 103, wherein the respective motions of the slave holder 104 and the master holder 103 are controlled by the surgeon's hands while performing similar motions at the front end of respective robotic arms.

Figure 1D schematically illustrates the situation when a corresponding hook is formed by the thread 102, by appropriately moving the master holder 103 after obtaining the needle 101 from the slave holder 104.

Figure 1E finally illustrates the situation during tightening of the respective knot, by pulling the needle 101 by means of the slave holder 104, while fixing the thread 102 by the master holder 103.

Figures 1F to 1J schematically illustrate respective process steps during the formation of a running suture, wherein the process steps shown in Figure 1F, 1G and 1H substantially correspond to the corresponding processes as shown and described with reference to Figures 1A to 1C.

In Figure 1I, after forming a corresponding loop, the position of the master holder 103 may be displaced in a direction substantially perpendicular to the drawing plane and then step 1J may be carried out in order to move the needle 101 through the tissue 151 at a desired longitudinal off-set, i.e. at an off-set perpendicular to the drawing plane, in order to provide a running suture, wherein the sequence of the steps 1F to 1J may be repeated as required.

Finally, a single knot may be tied, as was previously described with reference to Figures 1A to 1E. A corresponding analysis of suture-related surgical tasks may be obtained from respective data, for instance by observing laparoscopic surgery operation videos and the like.

In some illustrative embodiments, the analysis of the respective surgical task, on the basis of available data, may be followed by the establishment of corresponding equivalent process steps, i.e. motions of appropriately designed end-effectors, such as grippers or any other appropriate manipulators. For this purpose, the type and the degree of motional freedom of equivalent motions may be determined as are required, in order to fulfill the desired surgical task, such as the suture-related processes shown in Figures 1A to 1J. In some embodiments, the results obtained by establishing a respective set of equivalent motions, and the corresponding degrees of freedom, may then be used in order to set up an appropriate mechanical design for a robotic system, including an appropriate actuator system for accomplishing the required sequence of equivalent motions. In still other illustrative embodiments, available mechanical resources of robotic systems may be compared with respect to the requirements for the equivalent motions established, on the basis of the analysis of actual process steps, and an appropriate set of equivalent motions, having appropriate degrees of motional freedom, may be established in order to meet the respective capability of the available robotic system. For example, for the above-described surgical tasks, a set of equivalent motions may be established in which corresponding end-effector elements, such as the holders 103 and 104, perform a substantially planar motion, substantially without requiring an off-set with respect to the plane in question. For instance, the steps, as shown in Figures 1A to 1C and 1F to 1H, may be performed within a single plane, that is, the first and the second holders 103 and 104 may be moved within the drawing plane of Figure 1, without requiring, in this stage of the task, a motion perpendicular thereto. Consequently, in one illustrative embodiment, an equivalent motion for obtaining the process steps as shown in Figures 1A to 1C and Figures 1F to 1H, may include a corresponding rotational movement around a center of rotation, indicated as 105.

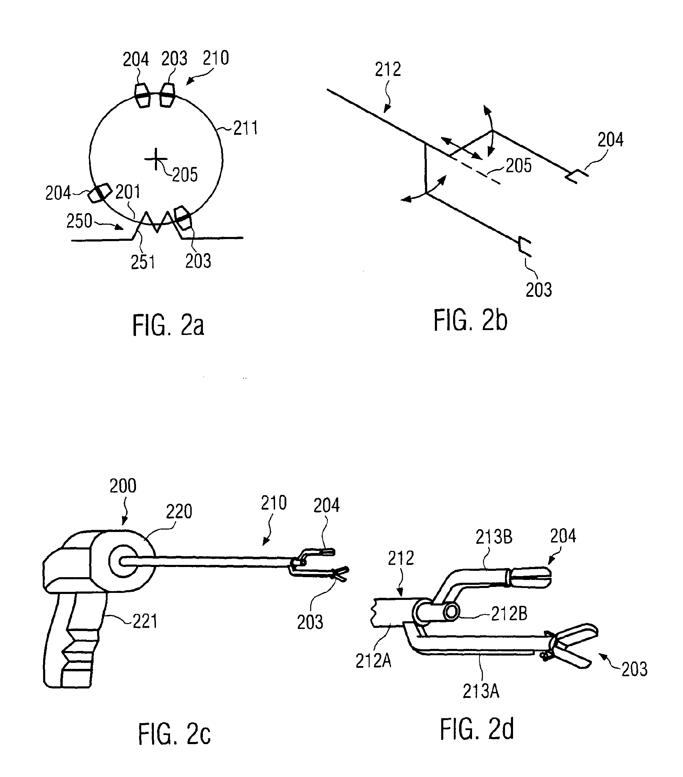

Figure 2A schematically illustrates a corresponding model of equivalent motions that may allow obtaining the process results as shown in Figures 1A to 1C and 1F to 1H. In Figure 2A an end-effector means 210 may be provided, which may, in one illustrative embodiment, comprise a first gripper 203 and a second gripper 204, which may move in a common plane, which, in the example shown, may be represented by the drawing plane of Figure 2A. In one illustrative embodiment, the first and the second grippers 203, 204, may both be rotatable around an axis of rotation 205, wherein both grippers 203 and 204 may perform, at least partially, a circular motion around the axis 205. Hereby, the axis 205 may be positioned with respect to tissue 251 such that the circle defined by the axis 205 and the circular motion of the grippers 203, 204, indicated as 211, corresponds to operation region 250, i.e. a region at which a respective suture is required, and substantially defines a tangent of the circle 211. Consequently, by using the circular motion of the grippers 203, 204, on the basis of the circle 211 having its center 205 appropriately positioned, the required degree of freedom of the end-effector system 210 is significantly reduced compared to a conventional robotic system and may readily be implemented by an efficient mechanical design.

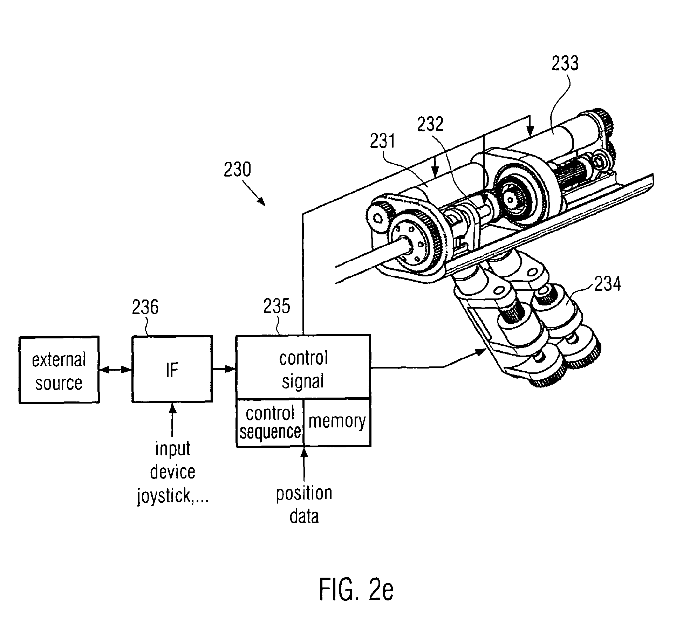

A further analysis of the motions of Figures 1A to 1E and 1F to 1J reveals that a linear motion, substantially perpendicular to the drawing plane of Figures 1 and 2, may be sufficient for accomplishing the complete surgical task shown in Figure 1. For instance, a linear off-set along the axis 205 may provide for the possibility of forming a hook for tying a knot or for providing a respective axial displacement for a running suture. That is, in one illustrative embodiment, the equivalent motions may be obtained on the basis of a system that is substantially restricted to the motions performed on a cylinder surface, thereby providing for a simple yet efficient mechanical design of the respective end-effector system 210.

Figure 2B schematically illustrates the principles of the mechanical design of an appropriate end-effector system, such as the system 210, wherein, in the illustrative embodiment shown, the synchronized motion, relative to each other, of the two grippers 203, 204, may be provided on the basis of co-axially arranged supports or holders 212, thereby providing for two linear and rotational degrees of freedom between the grippers 203, 204. Consequently, the system as schematically shown in Figure 2B, comprises the first and the second grippers 203, 204, each of which may be rotated around the axis 205, based on the respective holders 212, and are additionally moveable along the axis 205, wherein, additionally, one of the grippers, for instance the second gripper 204 may be moveable linearly with respect to the gripper 203, in order to provide for an appropriate exchange of the needle 210 between the grippers 203 and 204. For instance, a 30 mm range for the linear motion of the gripper 204 may be provided.

For example, in making a suture, as is, for instance illustrated in Figures 1A to 1E, the gripper 203 performs the first stage of the suturing process and, thereafter, the gripper 203 and the gripper 204 are positioned at 180 degrees against each other, in order to produce a complete hook for tying the knot. In this situation, a rotation of both grippers 203, 204, completes the hook. By a counter-clockwise rotation of the gripper 204 and gripping the needle 201, the corresponding task may be completed. Similarly, the running suture, as shown in Figures 1F to 1J, may be obtained by the equivalent motions provided by the system as shown in Figure 2B, by a respective linear off-set prior to each step for moving the needle 201 through the tissue 251. Consequently, by appropriately analyzing a respective suture-related surgical task and establishing a set of equivalent motions, the respective degree of motional freedoms may be determined, which may have to be provided by a respective mechanical system. In the embodiment shown in Figures 2A and 2B, a high reduction in the degrees of freedom is obtained by restricting the equivalent motions substantially to a cylinder surface, thereby defining the required degrees of freedom for a corresponding mechanical system. It should be appreciated that a more complex design may be used in order to provide for increased flexibility. For instance, the motions may be determined by rotational motions and linear motions, wherein the radius of the rotational motions may be variable. In other illustrative embodiments, available degrees of freedom of a mechanical system, for instance depending on the specific operational conditions, and the like, may be defined in advance and a respective sequence of equivalent motions for completing the surgical task under consideration, may be established, wherein a plurality of iterations may possibly be required. For instance, the different motions of the surgeon's hands may be analyzed, as is explained above, and a corresponding model of the motions may be developed, wherein individual steps of the model may represent corresponding equivalent motions that may be performed by a system having the pre-defined degrees of freedom. Consequently, for a pre-defined surgical task or a plurality of surgical tasks, the corresponding degrees of freedom of equivalent motions may be determined in order to establish design criteria for an appropriate mechanical system. Alternatively, for a given degree of freedom, appropriate equivalent motions may be established in order to accomplish one or more surgical tasks.

Figure 2C schematically illustrates the perspective view of a surgery device 200 for minimal invasive surgery, including a mechanical system based on the concept as described above, with reference to Figure 2B. That is, the device 200 may comprise a housing 220, including a handle 221, in which an appropriate actuator system (not shown) may be incorporated so as to effect the pre-defined sequence of motions on the basis of the required degree of freedom for an end-effector system such as the system 210. The system 210 may comprise the first gripper 203 and the second gripper 204, which may be supported by a pair of co-axial holders 212, i.e. by a first holder 212a supporting the gripper 203, and a holder 212b supporting the second gripper 204. As indicated in Figure 2B, the holders 212a and 212b are individually rotatable around the axis 205, thereby providing for the corresponding rotational or circular movement of the grippers 203, 204, as shown in Figures 2A and 2B.

Figure 2D schematically illustrates an enlarged view of a portion of the system 210. Moreover, the grip mechanisms of the grippers 203 and 204 may be actuated on the basis of respective cables 213a, 213b, wherein a corresponding spring mechanism may be provided in order to move corresponding gripper jaws from the closed position in the open position or vice versa. It should however be appreciated, that other appropriate actuator means and support assemblies may be used, depending on the overall configuration of the device 200 and its intended operation region. Moreover, the device 200 may comprise the actuator system configured to provide for a respective sequence of motions of the system 210, as will be described later on in more detail, in order to fulfill one or more specified surgical tasks, such as the suture-related processes described with reference to Figure 1.

Figure 2E schematically illustrates a perspective view of an actuator system 230, that may be incorporated in the device 200. The actuator system 230 may comprise a plurality of several actuators, i.e. for instance, electric motors or any other actuator devices, in combination with respective mechanical elements, for providing the respective rotational and linear motions, as is specified with reference to Figures 2A and 2B. Consequently, the actuator system 230 may comprise a first servo-actuator 231 coupled to the holder 212a for the gripper 203. Similarly, a corresponding servo-actuator 232, for the gripper 204, may be provided, and also a corresponding actuator 233 is provided, that allows a corresponding linear motion of the grippers 203, 204 and a relative linear motion, as was previously described. For instance, respective actuator elements 234 are provided so as to actuate the mechanisms 213a and 213b. In the embodiment illustrated in Figure 2E, the actuator elements may be provided in the form of appropriate electric servo-motors, while in other embodiments the actuator system 230 may include other appropriate actuating means, such as solenoids and the like. Moreover, the actuator system 230 may comprise a controller 235, which, in one embodiment, may be configured to provide a sequence of control signals to the respective actuator components 231, ... 234, in order to provide for a respective sequence of motions to complete the required surgical task. In still other embodiments, the controller 235 may provide respective drive signals on the basis of data received by an interface 236, wherein the interface 236 may represent an internal control component having implemented therein an appropriate means for generating or storing a respective instruction set that may cause the controller 235 to provide the appropriate control signals to the actuator components 231, ... 234. For example, the interface 236 may represent a micro-processor, including a data memory, which may have stored therein a corresponding instruction set that, upon execution, provides for appropriate instruction data, causing the controller 235 to provide the respective control signals. In still other illustrative embodiments, the interface 236 may represent a data communications interface for exchanging data with an external source (not shown) in which a respective set of data may be generated or retrieved in order to cause the controller 235, via the interface 236, to provide the appropriate signals. In still other illustrative embodiments, the interface 236 may be connected to any appropriate input devices, such as a joystick, a keyboard, various buttons, and the like, which may be operated by a user of the device 200, in order to retrieve a pre-defined sequence of motions so as to complete a specified surgical task.

In some illustrative embodiments, the actuator components 231, ... 234, or at least some components thereof, may be equipped with corresponding motor encoders or other position sensitive sensor elements, which may provide respective position data to the controller 235, which may be configured to provide the respective control signals on the basis of the position data. For example, upon requesting a specific action performed by one of the components 231, ... 234, the controller 235 may provide respective drive signals, for instance in the form of pulse width modulated power signals, for an electromotor, a number of pulses for a step motor, and the like, wherein the finally resulting motion of the motor axis may be determined on the basis of the corresponding position data so that an efficient feedback control mechanism may be established. In still other illustrative embodiments, the respective position data may be used, in addition or alternatively, as feed forward data, in order to provide an updated target value for a motion still to be performed. For instance, if the overall position of the device 200 may have been changed, for instance due to a movement of the surgeon's hand, a corresponding movement may be sensed by one or more position sensors and a corresponding recalculation of respective target values for one or more motions still to be performed, may be carried out by the controller 235, prior to actually delivering the corresponding drive signals to one or more of the components 231, ... 234. Consequently, an enhanced degree of accuracy may be accomplished, since, for instance, by using a feedback mechanism on the basis of appropriate position sensors such as motor encoders, and the like, the target position of the respective components of the end effecter system 210 may be achieved with high precision. Moreover, by using sensor data as additional information, respective target values for the predefined sequence of motions may appropriately be re-calculated so as to adapt the device 200 to a different situation, for instance, caused by an overall movement of the device 200, a movement of the operation region relative to the device 200 and the like.

In still other illustrative embodiments, the controller 235 may provide the respective control signals on the basis of additional process information, for instance on the basis of image data obtained by ultra sonic, x-ray, visual monitoring techniques and the like. For this purpose, respective data may be obtained via the interface 236 and may appropriately processed in the controller 235, when sufficient computational resources are provided in the controller 235. In still other illustrative embodiments, respective instructions for the controller 235 may be obtained via the interface 236, wherein corresponding process information may have been evaluated by an external source which may also generate an updated version of respective instructions to cause the controller 235 to provide respective updated control signals. For example, if the controller 235 may have implemented therein a corresponding set of data or instructions that may be executed upon demand, an updated version of the data or control instructions may be received via the interface 236 from an external source when corresponding process information may indicate the requirement for such an update. In some illustrative embodiments, a corresponding updating of the control sequence performed by the controller 235 may be accomplished during operation of the device 200 when performing the surgery thereby providing enhanced flexibility in adapting to a plurality of different situations. In still other illustrative embodiments, a corresponding control sequence may be reloaded into the controller 235 at any appropriate time so as to allow the device 200 to perform a specified surgical task at a time. In still other illustrative embodiments a plurality of control sequences may be implemented in the controller 235 and may be invoked by an operator by correspondingly actuating a respective input device via the interface 236.

In still other illustrative embodiments the interface 236 may be configured to enable a substantially real time data communication so as to allow the utilization of powerful external resources for establishing an appropriate control sequence which may be transferred to the actuator system 230 via the interface 236, wherein the controller 235 may act as an interface for translating the respective instructions into appropriate drive signals. Moreover, in some illustrative embodiments, the controller 235, when provided as a substantially independent unit, or in any external supervising control system, may have implemented therein a system having adaptive capabilities so as to "learn" and to more precisely select operational parameters on the basis of process information provided by any appropriate sources such as an operator, an image processing system and the like. For instance, parameters such as a tension of the thread and the force with which the suture needle may be pulled from one of the grippers 203, 204 or from the tissue 251, may be adjusted on the basis of additional process information, for instance, from visual or other inspection tools that provide information on the result of the surgical task. For example, respective parameters may be related to the resulting suture, e.g., the degree of leakage at the suture, determined on the basis of other examination procedures. In still other illustrative embodiments, the respective control system such as the controller 235 may be equipped with a self-learning system such as a neuronal network wherein for a predefined basic process sequence respective parameter values are established and asserted during a certain phase or during previous surgery tasks thereby continuously improving the efficiency and accuracy of the respective control sequence.

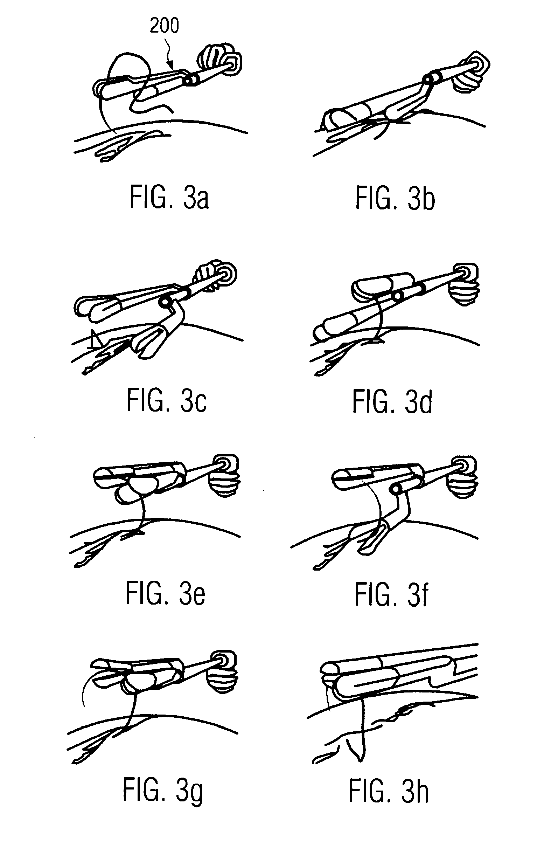

Figures 3A-3H schematically illustrate corresponding simulation results for performing a surgical task as, for instance, defined in Figure 1 on the basis of the device 200 wherein the simulation may be useful in determining appropriate equivalent motions in order to complete a specified surgical task. For instance, by establishing an appropriate sequence of motions on the basis of the degrees of freedom determined on the basis of a corresponding analysis, as previously described with respect to Figures 1, 2A and 2B, a corresponding model may be established for a respective mechanical system and the model may be used in order to simulate the efficiency of the respective virtual mechanical system. Based on the simulation results, the corresponding control sequence may be assessed and, may, if necessary, be reconfigured in order to enhance the efficiency thereof. For instance, Figure 3A schematically illustrates the step of moving the suture needle to an operating region and Figure 3B schematically illustrates the end of the first step in which a respective end effector such as the gripper 203 has moved the needle into the tissue and partially therethrough.

Figure 3C schematically illustrates the situation when the gripper is moved back while the other gripper grasps the needle. Figure 3D schematically illustrates the situation after advancing the needle on the basis of a corresponding rotation of the gripper.

Figures 3E-3H illustrate further stages of a corresponding process for removing the needle from the tissue and transferring the needle from one gripper to the other. Consequently, simulating a corresponding operational behaviour of the mechanical system on the basis of pre-established equivalent motions for accomplishing a specified surgical task may significantly enhance the efficiency in obtaining appropriate sets of required degrees of freedom and an appropriate mechanical system having the capability of performing the respective motions. Moreover, in situations when the mechanical capabilities of a given mechanical system may have to be examined with respect to the feasibility of a specific surgical task on an automatic basis, i.e. on a specific sequence of equivalent motions as is described above, a plurality of different sets of control sequences may conveniently be tested without requiring to actually implement a respective control sequence and perform the corresponding tasks.

As a result, the present invention provides a method for the mechanical design, the analysis of kinematic and dynamic aspects and the control architecture and algorithm implementation of a robotic system that may be used in endoscopic surgery. For this purpose, a typical surgical task, such as a suture related task, may be analyzed on the basis of surgeries performed by a human surgeon and/or on the basis of models, wherein an appropriate set of equivalent motions and the corresponding required degrees of freedom may be established from this information. Then, a corresponding mechanical design may be obtained and an appropriate control algorithm may be established which may be implemented into an actuator system or which may be implemented in any other appropriate control system and may be transferred in an appropriate manner to the actuator system which may then translate corresponding instructions into respective drive signals of the actuator components. Moreover, an intelligent or semi-intelligent surgery device from minimally invasive surgery is provided that comprises an actuator system having the capability to provide in an automated fashion a sequence of corresponding actuator actions for obtaining a sequence of motions for accomplishing a surgical task. Thus, upon activation of the mechanism, for instance, by a surgeon the burden imposed on the surgeon is significantly relaxed, for instance with respect to the angular positioning of the respective gripper elements as is the case in conventional endoscopic robot assisted systems. In one illustrative embodiment, any conventional curved suturing needle may be used in order to provide for a high degree of compatibility with conventional surgical instruments. Moreover, the method and devices of the present invention may be used during all stages of endoscopic surgery containing the fixation, making a single suture, tying a knot and forming a running suture. For example, by providing a joystick in an ergonomic handle of the device, the operation of the device is highly efficient and less demanding for the surgeon. By using a programmable microprocessor in the respective actuator system, the corresponding software of the device may be updated, for instance, in an automatic manner and/or by a systematic analysis of previously processed information in order to further enhance the efficiency of the device. On the basis of respective feedback sensors, the accuracy of the device may be even further enhanced. Thus, based on the present invention, precious time of the medical personnel may be saved, the operation may be simplified and the accuracy of suturing related tasks may be significantly improved, wherein the entire construction of the device may be maintained at a low level of complexity comparable to conventional devices. Moreover, the provision of a programmable behavior of the actuator system enables the updating and thus adaptation to various situations.

## Claims

1. An endoscopic surgery device for minimally invasive surgery, comprising:
end effector means configured to be movable in an operation region, and
an actuator system mechanically coupled to said end effector means and configured for automatically effecting a predefined sequence of actuator actions to actuate said end effector means for performing a predefined sequence of motions for forming at least a portion of a suture in said operation region.

2. The endoscopic surgery device of claim 1, wherein said end effector means comprises a gripping system.

3. The endoscopic surgery device of claim 2, wherein said gripping system comprises a first gripper and a second gripper.

4. The endoscopic surgery device of claim 3, wherein at least one of said first and second grippers is configured to be movable with respect to two or more degrees of freedom.

5. The endoscopic surgery device of claim 3, wherein at least one of said two or more degrees of motional freedom corresponds to a rotational motion around a specified axis.

6. The endoscopic surgery device of claim 5, wherein at least one of said two or more degrees of motional freedom corresponds to a linear motion along said specified axis.

7. The endoscopic surgery device of any of claims 1 to 6, further comprising a control unit operatively connected to said actuator system, said control unit being configured to control said sequence of actuator actions.

8. The endoscopic surgery device of claim 7, wherein said control unit is configured to obtain position data for controlling said sequence of actuator actions on the basis of said position data.

9. The endoscopic surgery device of claims 7 or 8, wherein said control unit is configured to control said sequence of actuator actions on the basis of said position data and at least one target value for at least some of the actions of said sequence.

10. The endoscopic surgery device of claim 9, wherein said actuator system comprises one or more position sensors for providing said position data.

11. The endoscopic surgery device of any of claims 7 to 10, wherein said control unit is configured to generate a plurality of control signals for driving said actuator system for effecting said predefined sequence of actuator actions.

12. The endoscopic surgery device of claim 11, wherein said control unit comprises means for updating said predefined sequence of actuator actions.

13. The endoscopic surgery device of claim 12, wherein said means for updating is configured to obtain process information related to an operation performed or to be performed by said endoscopic surgery device, and to update said predefined sequence of actuator actions on the basis of said process information.

14. The endoscopic surgery device of any of claims 1 to 13, wherein said gripping means is configured to support a suture needle.

15. The endoscopic surgery device of any of claims 1 to 14, further comprising a data communications interface for communication with an external source.

16. The endoscopic surgery device of claim 15, wherein said data communications interface is configured to provide real time data communication between said external source and said actuator system.

17. The endoscopic surgery device of any of claims 1 to 16, further comprising a housing including a handle, said handle being configured to provide for a one-hand operation of said surgery device.

18. The endoscopic surgery device of claim 3, further comprising a first holder coupling said first gripper to said actuator system and a second holder coupling said second gripper to said actuator system, said first and second holders being arranged in a coaxial relationship.

19. A method comprising:
determining a first sequence of motions of an end effector system of an endoscopic surgery device for a first specified surgery task, and
implementing a first control sequence in a control unit for controlling an actuator system for effecting said first sequence of motions.

20. The method of claim 19, wherein determining said first sequence of motions comprises analysing the entire motion of a surgical instrument required to complete said first specified surgery task and establishing a plurality of equivalent motions of said end effector system for completing said first specified surgery task using said surgical instrument.

21. The method of claim 20, wherein analysing said entire motion of the surgical instrument comprises determining a degree of freedom for each of said plurality of equivalent motions.

22. The method of any of claims 19 to 21, further comprising obtaining process data relating to said first specified surgery task accomplished by using said first control sequence and updating said first control sequence on the basis of said process data.

23. The method of claim 22, wherein updating said first control sequence is performed by said control unit.

24. The method of claim 22, wherein updating said first control sequence is performed by an external source via a data communications interface.

25. The method of any of claims 19 to 24, further comprising implementing a second control sequence in said control unit for controlling said actuator system for effecting a second sequence of motions of said end effector system for a second specified surgery task.
